# EUROPEAN PATENT APPLICATION

(11) **EP 4 516 919 A1**
(43) Date of publication of application: **05.03.2025**
(21) Application number: 23787692.5
(22) Date of filing: 11.04.2023
(51) Int. Cl.: C12P 13/04, C12N 5/10, C12N 9/10, C12N 9/80, C12N 15/63

(54) **PREPARATION METHODS FOR N-ACETYL-D-AMINO ACID, D-AMINO ACID, AND D-AMINO ACID DERIVATIVE**

(30) Priority: 12.04.2022 CN 202210381525
(71) Applicant: Mint Biotechnologies Co., Ltd., Hangzhou, Zhejiang 310012 (CN)
(72) Inventor: BI, Yanqi, Hangzhou, Zhejiang 310012 (CN); ZHANG, Kechun, Hangzhou, Zhejiang 310012 (CN)
(74) Representative: Gille Hrabal Partnerschaftsgesellschaft mbB Patentanwälte
(86) International application number: PCT/CN2023/087593
(87) International publication number: WO 2023/198048

(57) **Abstract**

The present invention relates to the technical field of genetic engineering and fermentation engineering, and specifically discloses a preparation method of an N-acetyl-D-amino acid. An L-amino acid and/or a D, L-amino acid are used as raw materials, the L-amino acid is converted into a D-amino acid under the action of an L-amino acid isomerase, and the D-amino acid is converted into an N-acetyl-D-amino acid under the action of an acyltransferase. General-use L-amino acid isomerase and acyltransferase are used to convert various amino acids into corresponding N-acetyl-D-amino acids. Raw materials used in the present method are low cost, toxic chemical racemization and high-cost chiral resolution steps are avoided, and the method is suitable for industrial processing and production. The prepared N-acetyl-D-amino acid can be further processed into a D-amino acid or a D-amino acid derivative.

## Description

### CROSS-REFERENCE TO RELATED APPLICATIONS

**The present disclosure claims priority to the prior application with the patent application No.** 202210381525.9 entitled "PREPARATION METHODS FOR N-ACETYL-D-AMINO ACID, D-AMINO ACID, AND D-AMINO ACID DERIVATIVE" and filed with the China National Intellectual Property Administration on April 12, 2022**, which is incorporated herein by reference in its entirety.**

### TECHNICAL FIELD

The present disclosure relates to the technical field of genetic engineering and fermentation engineering, in particular to a preparation method for an N-acetyl-D-amino acid, a D-amino acid and a D-amino acid derivative.

### BACKGROUND

Amino acids include two isomers, D-amino acids and L-amino acids, which exert different physiological functions in the living body. An excess of D-amino acids inhibits cell growth, and polypeptides containing D-amino acids are normally not or slowly hydrolyzed by peptidases. However, D-amino acids have good performance in the synthesis of β-lactam antibiotics and physiologically active peptides. For example, valine pyrethrin I synthesized by D-valine is 5,000-fold more active when compared to pyrethrin insecticides synthesized by L-amino acids. For another example, the sweetener Alitame containing one D-alanine has high sweetness but low calories, making it more suitable for patients with diabetes, obesity, and the like.

In recent years, the use of D-amino acids in the fields of medicines, agricultural chemicals, foods, and the like, has been widely and rapidly developed. The demand for D-amino acids is rapidly increasing across various fields, but the production of D-amino acids in the prior art is characterized by high unit price, low yield and individualization. Therefore, an efficient and universal preparation method is urgently needed to meet the actual demand. The existing production methods for D-amino acids include chemical resolution, chemical synthesis, biological fermentation, enzymatic method and the like, but all the methods are specific strategies established aiming at the synthesis of specific amino acids. The development cost of the production process for various types of D-amino acids is high, and the development processes are single in profit. Therefore, improvements are urgently needed. N-Acetyl-D-amino acid, used as an extremely important intermediate for producing D-amino acids, is also characterized by high unit price, low yield and individualization, and thus an efficient and universal preparation method is also urgently needed to meet the actual demand. Therefore, the present disclosure aims to establish a universal method for preparing an N-acetyl-D-amino acid in large quantity by using an L-amino acid, or a D,L-amino acid (i.e. mixture of a D-amino acid and an L-amino acid), or a mixture of the two as a starting material, and the N-acetyl-D-amino acid can be further used for preparing a D-amino acid and an amino acid derivative.

### SUMMARY

The technical problem to be solved by the present disclosure is to provide a universal method for preparing an N-acetyl-D-amino acid in large quantity by using an L-amino acid, or a D,L-amino acid, or a mixture of the two as a starting material, and the method is suitable for industrial processing.

The second technical problem to be solved by the present disclosure is to provide a universal method for preparing a D-amino acid in large quantity by using an L-amino acid, or a D,L-amino acid, or a mixture of the two as a starting material, and the method is suitable for industrial processing.

The third technical problem to be solved by the present disclosure is to provide a universal method for preparing a D-amino acid derivative in large quantity by using an L-amino acid, or a D,L-amino acid, or a mixture of the two as a starting material, and the method is suitable for industrial processing.

The fourth technical problem to be solved by the present disclosure is to provide a method for improving an N-acetyl-D-amino acid, or a D-amino acid, or a D-amino acid derivative.

The technical problem solved by the present disclosure is realized by adopting the following technical solutions:
Provided is a preparation method for an N-acetyl-D-amino acid, wherein an L-amino acid and/or a D,L-amino acid are used as a starting material. The L-amino acid is transformed into a D-amino acid under the action of an L-amino acid isomerase, and the D-amino acid is transformed into an N-acetyl-D-amino acid under the action of an acyltransferase.

Further, the transformation is completed in a bacterium or a fungus *in vivo,* or *in vitro* after the extraction of the L-amino acid isomerase and the acyltransferase.

Further, the L-amino acid and/or the D,L-amino acid are used as a starting material and as a substrate. A recombinant microorganism containing an L-amino acid isomerase-encoding gene and an acyltransferase-encoding gene is added for fermentation culture, and during the fermentation, the L-amino acid isomerase and the acyltransferase are generated by overexpression of the recombinant microorganism.

Further, the L-amino acid isomerase-encoding gene comprises one or more of ILEP, dadX, murI, ygeA, or alr, and the acyltransferase-encoding gene comprises one or two of Hpa3 and Hpa2.

Further, the construction of the recombinant microorganism by a genetic engineering method is included, and the genetic engineering method includes plasmid expression or genomic integration.

Further, the recombinant microorganism is constructed by the plasmid expression method, and the construction method is as follows: an L-amino acid isomerase-encoding gene and an acyltransferase-encoding gene are obtained by PCR amplification, the obtained genes are co-ligated to a plasmid vector containing an IPTG inducible promoter and transformed into a competent cell, and after sequencing, a recombinant vector is obtained; and the recombinant vector is transformed into a recipient microorganism to obtain the recombinant microorganism. Preferably, the plasmid vector is one or two of pZAlac and pZElac.

Further, the recombinant vector includes pZE-ILEP and pZE-Hpa3.

Further, the microorganism is selected from one or more of *Escherichia coli, Bacillus, Corynebacterium, Saccharomyces,* or *Streptomyces.*

Further, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

Further, during the fermentation, a fermentation temperature is 20 to 40 °C, and a rotation speed is 180-240 rpm.

Preferably, the fermentation temperature is 30-35 °C.

30 °C is preferred. 32 °C is preferred. 35 °C is preferred.

Preferably, the rotation speed is 220 rpm.

Further, during the fermentation, a culture medium adopted comprises starting materials in the following proportions: 11 to 13 g/L of M9 salts, 1 to 5 mM/L of magnesium sulfate, 0.1 to 0.5 mM/L of calcium chloride, 0.01 to 0.05 mg/mL of thiamine, 10 to 100 g/L of auxiliary material, 3 to 8 g/L of yeast powder, 1 to 3 mM/L of IPTG, and 30 to 60 µg/mL of kanamycin sulfate.

The auxiliary material is used for providing a carbon source. Glucose is preferred.

When the present disclosure is applied, calcium carbonate, calcium hydroxide, sodium carbonate, sodium bicarbonate, sodium hydroxide, potassium carbonate, or potassium hydroxide is adopted to adjust the pH. The fermentation product D-amino acid and pH regulator generate a corresponding salt to obtain D-amino acid calcium, D-amino acid potassium, D-amino acid sodium, or D-amino acid ammonium.

The present disclosure also relates to a preparation method for a D-amino acid, which is obtained by hydrolyzing the N-acetyl-D-amino acid obtained by the method described above, with the hydrolysis being carried out *in vitro.* Hydrolysis can be performed using various hydrolysis methods containing acids or bases such as hydrochloric acid, formic acid, sulfuric acid, potassium hydroxide, sodium hydroxide, or the like.

Provided is a preparation method for a D-amino acid derivative, wherein the N-acetyl-D-amino acid obtained by the method described above is hydrolyzed into a D-amino acid, and the D-amino acid is then transformed into a D-amino acid derivative.

The present disclosure further provides a recombinant microorganism for preparing a D-amino acid or its derivative, wherein the recombinant microorganism overexpresses endogenous or exogenous L-amino acid isomerase-encoding gene and acyltransferase-encoding gene;
preferably, the L-amino acid isomerase-encoding gene comprises one or more of ILEP, dadX, murI, ygeA, or alr, and the acyltransferase-encoding gene comprises one or two of Hpa3 and Hpa2;
preferably, the microorganism is selected from one or more of *Escherichia coli, Bacillus, Corynebacterium, Saccharomyces,* or *Streptomyces;* further preferably, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

The present disclosure further provides a recombinant DNA or biomaterial for preparing a D-amino acid or its derivative, wherein the recombinant DNA or biomaterial contains an L-amino acid isomerase-encoding gene and an acyltransferase-encoding gene;
preferably, the L-amino acid isomerase-encoding gene comprises one or more of ILEP, dadX, murI, ygeA, or alr, and the acyltransferase-encoding gene comprises one or two of Hpa3 and Hpa2;
the biomaterial is an expression cassette, a transposon, a plasmid vector, a phage vector, or a virus vector.

The present disclosure further provides use of the recombinant microorganism or the biomaterial for preparing a D-amino acid or its derivative described above, and the amino acid is a natural amino acid or unnatural amino acid.

The reaction mechanism of the present disclosure for preparing an N-acetyl-D-amino acid is as follows:

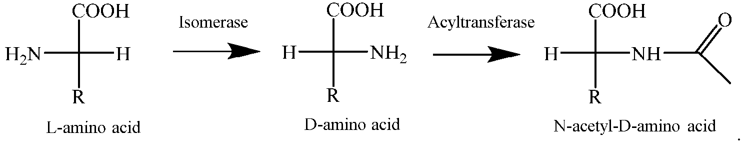

Beneficial effects: The preparation method for an N-acetyl-D-amino acid described herein is a universal preparation method. Various L-amino acids, or D,L-amino acids, or mixtures of the two can be directly used as substrates for fermentation, and various amino acids are transformed into corresponding N-acetyl-D-amino acids by utilizing universal L-amino acid isomerase and acyltransferase. The method utilizes low-cost starting materials, and avoids toxic chemical racemization and high-cost chiral resolution steps, making it suitable for industrial processing and production.

The preparation method for a D-amino acid described herein is a universal preparation method. Various L-amino acids can be directly used as substrates for fermentation, and N-acetyl-D-amino acids obtained by mass preparation can be hydrolyzed into corresponding D-amino acids *in vitro* after hydrolysis. The problem that D-amino acids are toxic to cells and thus have limited *in vivo* transformation is addressed, thereby better meeting the production needs of D-amino acids.

The preparation method for a D-amino acid derivative described herein is a universal preparation method and is suitable for industrial processing and production.

The present disclosure also effectively improves the transformation rate and the yield of N-acetyl-D-amino acids, D-amino acids, and D-amino acid derivatives.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the content described above of the present disclosure are encompassed within the protection scope of the present disclosure.

Unless otherwise stated, the starting materials and reagents used in the following examples are all commercially available products or can be prepared by using known methods. The scientific and technical terms used herein have the same meaning as commonly understood by those skilled in the art. Also, in order to better understand the present disclosure, the definitions and interpretations of the related terms are provided below.

Unless defined otherwise, or clear from the background, all technical and scientific terms used in the present disclosure have the same meaning as commonly understood by one of ordinary skill in the art to which the present disclosure belongs.

As used herein and unless otherwise specified, the term "L-amino acid" is all of the basic amino acids of all L configurations. The term "D-amino acid" refers to all of the basic amino acids of D configuration. The term "N-acetyl-D-amino acid" refers to all D configuration basic amino acids that are acetylated; the "amino acid" in the present disclosure includes natural amino acids such as glycine, phenylalanine, serine, and the like; and unnatural amino acids such as norvaline and norleucine.

The term "amplification" refers to the enhancement of the intracellular activity of one or more enzymes encoded by appropriate DNA within microorganisms, achieved, for example, by increasing gene copy numbers, using strong promoters, or employing genes that encode suitable enzymes with high activity, and selectively combining these methods.

The term "wild" refers to an object that can be found in nature.

Unless specifically stated otherwise, the terms "first", "second", and the like, do not denote any order or importance, but rather the terms first, second, and the like are used to distinguish one object from another.

As used herein and unless otherwise specified, when the term "about" modifies a measurable value such as an amount, a period of time, etc., it refers to a variation of ± 10%, preferably ±5%, more preferably ±1% and even more preferably ±0.1% from the given value, so long as such variation is suitable for implementing the method disclosed herein.

As described above, one of the objects of the present disclosure is to provide a preparation method for a D-amino acid and an N-acetyl-D-amino acid, which is safe and environmentally friendly and has a high yield.

Some embodiments of the present disclosure disclose a preparation method for a D-amino acid and an N-acetyl-D-amino acid, in which L-amino acids are added to a culture medium as substrates, and the fermentation substrates are fermented or biotransformed *in vitro* by bacteria or fungi.

The DNA polymerase Phanta Max Super-Fidelity DNA Polymerase and the non-ligase-dependent single-fragment quick cloning kit ClonExpress^{®}II One Step used in the embodiments of the present disclosure are purchased from Nanjing Vazyme Biotech Co., Ltd. LB medium composition: 10 g/L of tryptone, 5 g/L of yeast powder, 10 g/L of sodium chloride, and 1.5% of agar powder added into a solid culture medium.

Antibiotic concentration: 100 µg/mL of ampicillin.

Fermentation culture medium components:

| Component | Concentration |
|---|---|
| M9 salts | 11.2 g/L |
| Magnesium sulfate | 1 g/L |
| Calcium chloride | 0.1 g/L |
| Thiamine | 0.01 g/L |
| D-glucose | 40 g/L |
| Yeast powder | 5 g/L |
| IPTG | 1 mM/L |
| Kanamycin sulfate | 50 µg/mL |

The detection method for D-amino acids and N-acetyl-D-amino acids is as follows: D-amino acids and N-acetyl-D-amino acids are quantitatively detected using HPLC-RID equipped with a Bio-Rad Column (1250140 Aminex HPX-87H Column 300 × 7.8 mm) and an Agilent C18 Column.

The recombinant vectors used in some examples are constructed as follows:
Primers were designed based on ILEP and Hpa3 sequences published by NCBI, respectively: ILEP_F(ATGGGTAAATTAGACAAAGCGTCTAAATTAATTGATGAAG)/ ILEP_R(TTATTACCAGCCAATTTTACCTGTATCCTTGGG) Hpa3_F(AAAGGTACCATGAAAAAGACCCCAGACCC) / Hpa3_R(AGAAAGCTTTTAGTATCCGTTTCTCTTGT)

ILEP (L-amino acid isomerase) and Hpa3 (acyltransferase) genes were obtained by PCR amplification, and were respectively ligated to a vector pZElac comprising an IPTG inducible promoter by a non-ligase-dependent single-fragment quick cloning kit. Then, the vector was transformed into BW25113 competent cells, and a kanamycin sulfate resistance plate was coated with the cells for culturing overnight. Positive clones were selected for sequencing verification, and the correct recombinant vectors were named pZE-ILEP and pZE-Hpa3 separately.

The nucleotide sequence of ILEP is set forth in SEQ ID NO: 1. The nucleotide sequence of Hpa3 is set forth in SEQ ID NO: 2.

Specific information of proteins encoded by the genes used herein is shown in the following table. In the context of the present application, the enzymes mentioned comprise mutants which retain enzymatic activity, and the mutants have an amino acid sequence having at least 98% or at least 99% sequence identity to the amino acid sequences represented by the ACCESSION numbers in the table below; for example, ILEP refers to an enzyme having an amino acid sequence (M1GRN3) encoded by the nucleotide sequence of SEQ ID NO: 1; the enzymes also comprise mutants which retain L-amino acid isomerism activity, and the mutants have an amino acid sequence having at least 98% or at least 99% sequence identity to the amino acid sequence encoded by ID NO: 1.

| Gene | ACCESSION number of encoded protein |
|---|---|
| ILEP | M1GRN3 |
| dadX | NP_415708.1 |
| murI | NP_418402.2 |
| ygeA | NP_417317.1 |
| alr | NP_418477.1 |
| Hpa3 | AJU42942.1 |
| Hpa2 | NP_015519.1 |

### Example 1

The recombinant plasmids pZE-ILEP and pZE-Hpa3 were electroporated into *Escherichia coli* BW25113 to obtain recombinant bacteria, and the single colonies of the recombinant bacteria were separately inoculated into a 2 mL LB liquid medium containing 50 µg/mL of kanamycin sulfate and cultured overnight at 37 °C and 220 rpm (about 14 h). The bacteria were transferred to a 100 mL Erlenmeyer flask containing 5 mL of fermentation culture medium at an initial OD of 0.05, and cultured at 30 °C and 220 rpm. 0.5 g CaCO₃ was added to each fermentation flask to adjust the pH of the fermentation broth, while 0.2 g of the corresponding L-type amino acid was added as a substrate to be catalyzed. After 24 h of culture, the fermentation broth was collected, and the concentration of N-acetyl-D-amino acid was detected (see Table 1). In this example, CaCO₃ was used to adjust the pH of the fermentation broth.

**Table 1 Detection result table**

| | Substrate → product | Yield (g/L) | Transformation rate (%) |
|---|---|---|---|
| 1 | L-Ala→N-D-Ala | 7.40±0.11 | 74.04% |
| 2 | L-Ser→N-D-Ser | 1.69±0.61 | 16.92% |
| 3 | L-Phe→N-D-Phe | 9.97±0.04 | 99.67% |
| 4 | L-Val→N-D-Val | 5.34±0.51 | 53.42% |
| 5 | L-Met→N-D-Met | 6.95±0.07 | 69.53% |
| 6 | L-Asp→N-D-Asp | 0.14±0.07 | 1.44% |
| 7 | L-pGly→N-D-pGly | 6.42±0.17 | 64.20% |
| 8 | L-Arg→N-D-Arg | 10.22±0.05 | 10.22% |
| 9 | L-Gln→N-D-Gln | 1.35±0.32 | 13.50% |
| 10 | L-Leu→N-D-Leu | 7.39±0.64 | 73.90% |
| 11 | L-Ieu→N-D-Ileu | 1.46±1.21 | 14.60% |
| 12 | L-Gly→N-D-Gly | 2.25±0.77 | 22.50% |
| 13 | L-Glu→N-D-Glu | 0.30±0.28 | 2.97% |
| 14 | L-norleucine→N-D-norleucine | 6.98±0.97 | 69.8% |
| 15 | L-norvaline-N-D-norvaline | 7.61±1.44 | 76.1% |

The N-acetyl-D-amino acids obtained in the example can be further hydrolyzed into the corresponding D-amino acids, which are further used for preparing D-amino acid derivatives. The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

### Sequence information:

NO: 1
Length: 1353
Type: DNA
Species: *Lactobacillus buchneri* 
atgggtaaat tagacaaagc gtctaaatta attgatgaag aaaacaagta ttatgcacgt 60

tcggcgcgca tcaattacta caatctggta attgaccacg cacacggggc aaccttagtg 120

gatgtcgacg gcaataaata cattgatttg ctggcaagcg catctgcaat taacgttggc 180

cacacccatg aaaaagtcgt caaggcgatc gccgatcaag ctcaaaaatt aattcattac 240

actcccgctt attttcacca tgttcctgga atggaacttt ccgaaaagtt agcgaaaatt 300

gcgccgggaa attcaccgaa aatggttagt ttcggtaact ccggctcaga cgcaaacgat 360

gcaattatca agtttgcgag agcctatact ggccgtcaat acattgtttc ctatatgggt 420

tcctatcatg ggtcaactta tggttcgcaa accttatcag gaagtagtct taacatgaca 480
 
cgtaaaattg ggccaatgct tccaagcgtg gtgcacgttc cgtatccaga ttcatatcga 540

acctatccgg gagaaaccga gcacgacgtt tcactaaggt attttaacga attcaagaag 600

ccattcgaat cattcttacc cgctgatgaa actgcgtgtg tcttgattga accaattcaa 660

ggcgatggcg gcatcatcaa agcccctgaa gaatatatgc agttagttta taaattctgt 720

catgaacacg ggatcctgtt tgcgattgat gaagtcaatc aaggactcgg ccggaccggt 780

aagatgtggg caatccaaca attcaaagac atcgaacctg atttaatgtc tgtcggtaag 840

tcgctggcgt ctggaatgcc ccttagtgca gtgattggta agaaagaggt catgcagagt 900

ttggatgcac ctgcgcacct gtttaccact gccggcaatc cggtttgttc cgcagcttcg 960

ctggctacct tggatgtgat cgaatatgaa ggcttggttg aaaaatcggc caccgatggt 1020

gcatacgcca aacaacgttt cttagagatg cagcaacgcc atcccatgat tggtgatgtc 1080

cgaatgtggg gcctcaatgg tggaatcgaa ttggtcaagg atcctaagac caaggaaccc 1140

gacagtgacg ctgccaccaa agtgatctac tatgcctttg cacacggcgt cgttatcatc 1200

actttggcag gcaatattct gcgtttccag ccgccgttgg tcattccaag agagcaactc 1260

gaccaagcgc ttcaagtgct tgacgacgcg tttaccgcag ttgaaaatgg tgaggttacc 1320

attcccaagg atacaggtaa aattggctgg taa 1353
NO: 2
Length: 540
Type: DNA
Species: *Saccharomyces cerevisiae*

atgaaaaaga ccccagaccc atctccacct tttgcaagca cgaagaacgt aggcatgtca 60

aacgaagaac cggaaaaaat ggttaacgat cgaattgtgg tgaaggccat cgagccaaaa 120

gatgaggaag cctggaacaa actgtggaag gagtatcagg gtttccaaaa aacggttatg 180

cctccggaag tagccaccac taccttcgca aggtttatag accctacggt caaactatgg 240

ggtgctctag cctttgacac cgagaccggc gatgcaattg gctttgcaca ctacctgaac 300

catttgacgt cgtggcatgt cgaagaagtt gtctacatga atgacttata tgtcactgaa 360

cgcgcaagag tcaaaggcgt aggtagaaag ctcatagaat ttgtatacag ccgtgccgat 420

gagctcggta cgcctgcagt gtactgggtt acagaccatt acaatcaccg cgcacagttg 480

ctgtacacca aagtggctta caagacagat aaggttcttt acaagagaaa cggatactaa 540

## Claims

1. A preparation method for an N-acetyl-D-amino acid, wherein an L-amino acid and/or a D,L-amino acid are used as a starting material, the L-amino acid is transformed into a D-amino acid under the action of an L-amino acid isomerase, and the D-amino acid is transformed into an N-acetyl-D-amino acid under the action of an acyltransferase; and the amino acids are natural amino acids or unnatural amino acids.

2. The preparation method for an N-acetyl-D-amino acid as claimed in claim 1, wherein the transformation is completed in a bacterium or a fungus *in vivo,* or *in vitro* after the extraction of the L-amino acid isomerase and the acyltransferase.

3. The preparation method for an N-acetyl-D-amino acid as claimed in claim 1, wherein the L-amino acid and/or the D,L-amino acid are used as a substrate; a recombinant microorganism containing an L-amino acid isomerase-encoding gene and an acyltransferase-encoding gene is added for fermentation culture; and during the fermentation, the L-amino acid isomerase and the acyltransferase are generated by overexpression of the recombinant microorganism.

4. The preparation method for an N-acetyl-D-amino acid as claimed in claim 3, wherein the L-amino acid isomerase-encoding gene comprises one or more of ILEP, dadX, murI, ygeA, or alr, and the acyltransferase-encoding gene comprises one or two of Hpa3 and Hpa2.

5. The preparation method for an N-acetyl-D-amino acid as claimed in claim 3, comprising the construction of the recombinant microorganism by a genetic engineering method, wherein the genetic engineering method comprises plasmid expression or genomic integration.

6. The preparation method for an N-acetyl-D-amino acid as claimed in claim 5, wherein the recombinant microorganism is constructed by the plasmid expression method, and the construction method is as follows: an L-amino acid isomerase-encoding gene and an acyltransferase-encoding gene are obtained by PCR amplification, the obtained genes are co-ligated to a plasmid vector containing an IPTG inducible promoter and transformed into a competent cell, and after sequencing, a recombinant vector is obtained; and the recombinant vector is transformed into a recipient microorganism to obtain the recombinant microorganism.

7. The preparation method for an N-acetyl-D-amino acid as claimed in claim 6, wherein the recombinant vector comprises pZE-ILEP or pZE-Hpa3.

8. The preparation method for an N-acetyl-D-amino acid as claimed in claim 3, wherein the microorganism is selected from one or more of *Escherichia coli, Bacillus, Corynebacterium, Saccharomyces,* or *Streptomyces.*

9. The preparation method for an N-acetyl-D-amino acid as claimed in claim 3, wherein the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

10. The preparation method for an N-acetyl-D-amino acid as claimed in claim 3, wherein during the fermentation, a fermentation temperature is 20 to 40 °C, and a rotation speed is 180-240 rpm.

11. The preparation method for an N-acetyl-D-amino acid as claimed in claim 3, wherein during the fermentation, a culture medium adopted comprises starting materials in the following proportions: 11 to 13 g/L of M9 salts, 1 to 5 g/L of magnesium sulfate, 0.1 to 0.5 g/L of calcium chloride, 0.01 to 0.05 mg/mL of thiamine, 10 to 100 g/L of auxiliary material, 3 to 8 g/L of yeast powder, 1 to 3 mM/L of IPTG, and 30 to 60 µg/mL of kanamycin sulfate.

12. A preparation method for a D-amino acid, wherein an N-acetyl-D-amino acid is obtained by the method as claimed in any one of claims 1-11, followed by hydrolysis to obtain a D-amino acid, with the hydrolysis being carried out *in vitro.*

13. A preparation method for a D-amino acid derivative, wherein an N-acetyl-D-amino acid is obtained by the method as claimed in any one of claims 1-11, followed by hydrolysis to obtain a D-amino acid, and the D-amino acid is then transformed into a D-amino acid derivative.

14. A recombinant microorganism for preparing a D-amino acid or its derivative, wherein the recombinant microorganism overexpresses endogenous or exogenous L-amino acid isomerase-encoding gene and acyltransferase-encoding gene;
preferably, the L-amino acid isomerase-encoding gene comprises one or more of ILEP, dadX, murI, ygeA, or alr, and the acyltransferase-encoding gene comprises one or two of Hpa3 and Hpa2;
preferably, the microorganism is selected from one or more of *Escherichia coli, Bacillus, Corynebacterium, Saccharomyces,* or *Streptomyces;* further preferably, the microorganism is selected from one or more of *Escherichia coli, Bacillus subtilis, Bacillus megaterium, Bacillus amyloliquefaciens, Corynebacterium glutamicum, Saccharomyces cerevisiae, Candida utilis,* or *Pichia pastoris.*

15. A recombinant DNA or biomaterial for preparing a D-amino acid or its derivative, wherein the recombinant DNA or biomaterial contains an L-amino acid isomerase-encoding gene and an acyltransferase-encoding gene;
preferably, the L-amino acid isomerase-encoding gene comprises one or more of ILEP, dadX, murI, ygeA, or alr, and the acyltransferase-encoding gene comprises one or two of Hpa3 and Hpa2;
the biomaterial is an expression cassette, a transposon, a plasmid vector, a phage vector, or a virus vector.

16. Use of the recombinant microorganism as claimed in claim 14 or the biomaterial as claimed in claim 15 for preparing a D-amino acid or its derivative, wherein the amino acid is a natural amino acid or unnatural amino acid.
